# EUROPEAN PATENT SPECIFICATION

(11) **EP 3 532 144 B1**
(45) Date of publication and mention of the grant of the patent: **09.12.2020**
(21) Application number: 17777925.3
(22) Date of filing: 06.10.2017
(51) Int. Cl.: A61M 25/09, A61J 15/00

(54) **TUBE DEVICE FOR ADMINISTERING A MEDICAL FLUID TO A PATIENT**
TUBE DEVICE FOR ADMINISTERING A MEDICAL FLUID TO A PATIENT
DISPOSITIF TUBULAIRE DESTINÉ À ADMINISTRER UN FLUIDE MÉDICAL À UN PATIENT

(30) Priority: 26.10.2016 EP 16195679
(43) Date of publication of application: 04.09.2019
(73) Proprietor: Fresenius Kabi Deutschland GmbH, 61352 Bad Homburg (DE)
(72) Inventor: AMON, Barbara, 65510 Idstein (DE); SCHÄFER, Benjamin, 35644 Hohenahr (DE)
(74) Representative: Fresenius Kabi Deutschland GmbH
(86) International application number: PCT/EP2017/075472
(87) International publication number: WO 2018/077592

(56) References cited:
- EP-A1- 1 774 985
- US-A1- 2010 241 104

## Description

The invention relates to a tube device for administering a medical fluid to a patient according to the preamble of claim 1.

A tube device of this kind, in particular an enteral feeding device, comprises a tube and a guide device having a guide wire which is insertable into the tube for introducing the tube into a body opening of a patient.

A tube device of this kind may for example be suited to be introduced through the nose or the mouth of a patient to enter via the esophagus into the stomach of the patient in order to deliver an enteral feeding solution to the gastro-intestinal tract of the patient. The tube herein is soft and flexible and constitutes the delivery line through which the feeding solution is entered into the stomach of the patient. The guide device, with its guide wire, serves to place the tube in the patient, wherein for the placement the guide wire is inserted into the tube and is sufficiently rigid such that it allows to push the tube towards the body opening of the patient.

An enteral feeding device comprising a tube and a guide wire is for example disclosed in US 5,242,429.

When placing the tube of a tube device in a body opening it must be made sure that the tube in fact is inserted into the body opening to which access is desired and not into another opening. For example, if the tube is to be introduced into the stomach of a patient, it must be made sure that the tube in fact reaches the stomach and does not extend towards another body opening such as the lung.

For this, US 8,696,567 proposes a testing method within which a sample of body fluid taken from the body opening into which the tube extends is taken once the tube is placed in the patient. For example, body fluid contained in the stomach of a patient is known to contain enzymes such as human gastric lipase (HGL). By subjecting a testing device having a pH indicating surface impregnated with a suitable ester compound to the body fluid it can be checked, according to a pH indication on the pH indicating surface, whether human gastric lipase is present in the body fluid.

US 8,696,567 proposes a method requiring to take a sample of body fluid for example by aspiration, which then can be checked for the presence of particular substances outside of the human body (ex vivo).

Another approach to confirm correct placement of an enteral feeding tube uses imaging techniques to visualize or track the enteral feeding tube within the patient's body, which however is cumbersome and requires substantial technical equipment, which may not always be available at any time. A tube device according to the preamble of claim 1 is known from EP1774985.

It is an object of the instant invention to provide a tube device which allows, in an easy and reliable manner, to confirm correct placement of a tube in a patient's body.

This object is achieved by means of a tube device comprising the features of claim 1.

Accordingly, the guide wire comprises a probe device for testing or obtaining a sample of a body fluid present in the body opening.

Hence, the guide wire comprises a probe device which allows for confirming whether the tube has entered the body opening in which it shall be placed. The probe device herein may be constituted to (directly) test a sample of a body fluid, for example with regard to the presence of particular substances in the body fluid, or to obtain a sample of the body fluid which then may be taken from the tube by pulling the guide wire out of the tube in order to then test the example outside of the human body (ex vivo).

By providing a probe device on the guide wire an easy confirmation of the correct placement of the tube in the patient's body becomes possible, without the need for taking a sample by aspiration, i.e. without the need to aspire body fluid through the tube (for example using a syringe). Rather, by means of the probe device on the guide wire body fluid may be tested within the body opening itself or a (very) small volume sample of body fluid may be delivered from the body opening towards the outside of the body to test the body fluid outside of the body.

Confirmation of correct placement of the tube device hence is easily and reliably possible anytime and anywhere, without the need for extensive training of personnel and without requiring extensive technical equipment.

In one example, the probe device is placed at a distal end of the guide wire, the guide wire being inserted into the tube with the distal end. When placing the tube in the patient, the guide wire extends within the tube and reaches, with its distal end, towards a distal end of the tube. The distal end of the guide wire hence is placed at or close to the distal tube end which, upon correct placement in the body, reaches into the body opening. The probe device at the distal end of the guide wire hence may come into contact with the body fluid contained in the body opening, such that the body fluid may be tested or a small sample of body fluid may be taken to deliver it towards the outside.

In one example, the tube comprises at least one opening constituted to allow body fluid to enter into the tube. The probe device of the guide wire received within the tube in this case interacts with the body fluid within the tube, for example to take a sample of the body fluid or to perform a testing of the body fluid.

According to the invention, the opening of the tube is not opened at all times, but may selectively be opened by moving the guide wire within the tube. The guide wire in a first position may be placed in the tube to close off the opening such that body fluid is prevented from entering into the tube. The guide wire assumes this first position while placing the tube in the patient. In a second position, in contrast, the guide wire may be placed in the tube to uncover the opening such that body fluid is allowed to enter into the tube to interact with the probe device. Into this second position the guide wire is moved after the tube (presumably) has reached its position in the patient. In this way it is made sure that the opening of the tube is opened only once the tube has entered the body opening which it shall rest in, such that body fluid is tested or a sample is taken only within the body opening that the tube has reached, but not elsewhere.

For exemplary purposes, the tube, at its distal tube end, may comprise an exit opening through which the probe device of the guide wire may be moved to exit from the tube. For placing the tube in the patient's body the probe device is retracted into the tube and hence is concealed within the tube. Once the body opening is reached, the probe device may be moved outside of the tube such that it protrudes from the tube and a test on the body fluid in the opening may be performed or a sample may be taken. Once the test has been performed or the sample has been taken, the guide wire with the probe device is retracted back into the tube and may be removed from the tube to perform a visual inspection or a testing.

In one embodiment, the probe device comprises at least one indicator device which is constituted, upon interaction with body fluid, to indicate at least one property of the body fluid. The indicator device, in one example, may be constituted to indicate the presence of at least one substance in the body fluid.

In one embodiment, the indicator device may comprise an ester compound and a pH-sensitive substance for obtaining an indication of the presence of a hydrolytic enzyme, in particular human gastric lipase, in the body fluid. This is in particular suitable for confirming the correct placement of the tube in the stomach of a patient. It is known that the stomach fluid contains, amongst others, enzymes such as human gastric lipase, which may differentiate body fluid in the stomach from body fluids in other body openings of the patient. Human gastric lipase may be selectively tested for - as it is described in US 8,696,567, for example by impregnating a pH sensitive surface by an ester compound. Upon contact with a body fluid containing human gastric lipase acid is released by hydrolysis and a change in pH is registered on the pH sensitive surface.

Suitable ester compounds to be used in such an indicator device for example may be tributyrin, tricaprylin, triacetine, n-butyric acid methyl, benzyl butyrate, ethyl levulinate, ethylacetate or tristerain, as described in US 8,696,567.

In another embodiment, the probe device may - in addition or alternatively - comprise a probing chamber for receiving a sample of body fluid. By such a probe device a small volume sample may be taken and may be removed from the tube by pulling the guide wire out of the tube such that a testing outside of the body (ex vivo) is possible.

In one embodiment, the guide wire comprises an insertion piece movable with respect to the probing chamber for delivering body fluid into the probing chamber. The probing chamber, in this embodiment, may for example by slidable with respect to the insertion piece, which may be fixedly connected to the guide wire. By moving the insertion piece relative to the probing chamber when withdrawing the guide wire from the tube, body fluid may be drawn into the probing chamber, such that the sample taken this way may be removed from the tube by pulling the guide wire out of the tube.

In another embodiment, the probe device may comprise a sponge element for storing body fluid. Body fluid, which makes contact with the probe device, hence is received and stored in the sponge element, such that it may be safely removed from the tube.

In yet another embodiment, the probe device may comprise at least one deformable concealing element which in a non-deformed state conceals a reception opening. In the reception opening, for example a sponge element is placed. The reception opening may be uncovered by deforming the concealing element such that body fluid may enter into the reception opening and may be received for example in the sponge element placed in the reception opening.

The embodiments described before may be used alternatively or in addition. For example, it is conceivable that a probe device comprises both in indicator device for performing a testing of a body fluid within the body opening and, in addition, a probing chamber to take a sample of body fluid for an additional testing outside of the patient's body.

The idea of the invention shall subsequently be described in more detail with reference to the embodiments illustrated in the figures. Herein:
- Fig. 1: shows a schematic drawing of a patient and a tube device placed transnasally in the stomach of the patient;
- Fig. 2: shows a schematic view of a distal end of a tube device;
- Fig. 3A: shows a schematic view of an embodiment of a probe device placed on a guide wire within a tube of the tube device;
- Fig. 3B: shows the probe device in contact with body fluid;
- Fig. 4A: shows a schematic view of an exemplary embodiment of a probe device arranged on a guide wire, in a position within a tube;
- Fig. 4B: shows the probe device in a state in which it is moved to protrude from the tube;
- Fig. 5A: shows a schematic view of another embodiment of a probe device arranged on a guide wire;
- Fig. 5B: shows the probe device in a state in which it is in contact with body fluid;
- Fig. 6A: shows a schematic view of yet another embodiment of a probe device arranged on a guide wire;
- Fig. 6B: shows the probe device in a state in which an insertion piece is moved in order to take a sample of body fluid;
- Fig. 6C: shows the probe device when moving the guide wire out of the tube;
- Fig. 7A: shows a schematic view of yet another embodiment of a probe device;
- Fig. 7B: shows the probe device in a state suited to take a sample;
- Fig. 8A: shows a schematic view of yet another embodiment of a probe device;
- Fig. 8B: shows the probe device in a state for taking a sample;
- Fig. 9A: shows a schematic view of yet another exemplary embodiment of a probe device; and
- Fig. 9B: shows the probe device in a state in which it is moved out of the tube to protrude from the tube in order to take a sample.

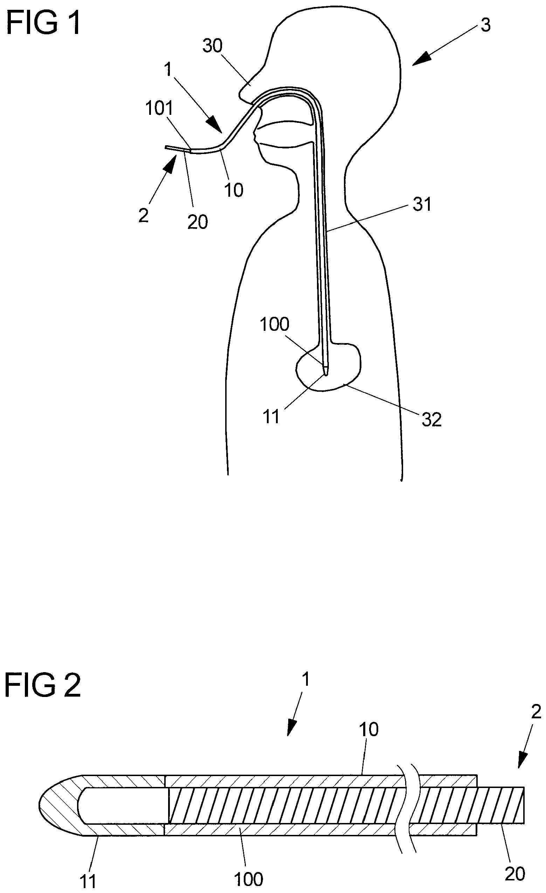

Fig. 1 shows, in a schematic drawing, a tube device 1 constituted as an enteral feeding device having a tube 10 and a guide device 2 inserted, with a guide wire 20, into the tube 10 for placing the tube 10 through the nose 30 and the esophagus 31 in the stomach 32 of a patient 3.

The tube device 1 serves to provide an enteral feeding into the gastro-intestinal tract of the patient 3. For this, upon correct placement of the tube 10 reaching into the stomach 32 of the patient 3 and upon removing the guide device 2 from the tube 10 a feeding system may be connected to a connector at a proximal end 101 of the tube 10 such that an enteral feeding solution may be delivered through the tube 10 into the stomach 32 of the patient 3.

The placement of the tube 10 in the patient 3 is guided by the guide device 2 such that the tube 10 may be inserted through the nose 30 and the esophagus 31 into the stomach 32. Upon placement of the tube 10 in the patient 3 it, however, must be made sure that the tube 10 correctly reaches into the stomach 32 with its distal end 100 such that feeding may take place directly into the stomach 32. In particular, it must be avoided that the tube 10 erroneously is introduced into another body opening such as for example the lung.

Fig. 2 shows a schematic, enlarged view of a distal part of the tube device 1 with its tube 10 and the guide device 2 inserted therein. The guide wire 20 of the guide device 2 is bendable, but sufficiently rigid such that it allows pushing the tube 10 into the patient 3, as it is commonly known in the art. The guide wire 20 may for example comprise a coiled helical spring encompassing a wire element and having a sufficient rigidity such that the soft, resilient tube may be guided through the esophagus 31 towards the stomach 32.

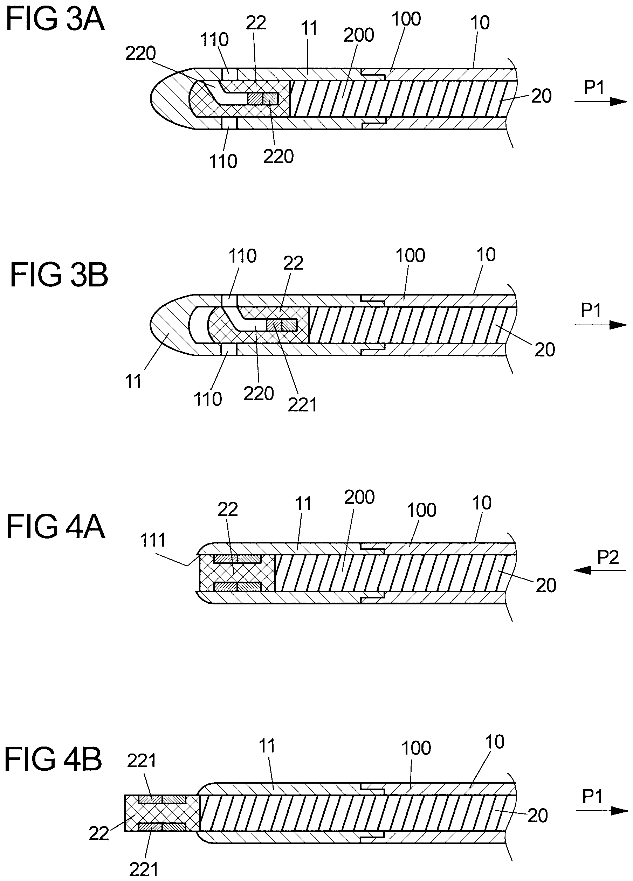

In order to confirm that the tube 10 has been correctly inserted into the stomach 32 of a patient 3, in an embodiment illustrated in Fig. 3A and 3B the guide wire 20 comprises, at its distal end 200, a probe device 22 serving to test body fluid contained in the body opening the tube 10 has entered.

The probe device 22 at the distal end 200 of the guide wire 20 is received within the tube 10 when placing the tube 10 inside the patient 3. The probe device 22 herein comes to rest within a tip element 11 connected to the tube 10 at its distal tube end 100, the tip element 11 for example being formed from a rigid material (in comparison to the soft, resilient tube 10) and having a rounded tip such that it may be guided in an easy way for example through the esophagus 31.

When placing the tube 10 in the patient 3, the tube 10 is pushed into the patient 3 by pushing on the guide wire 20. In this state the probe device 22 is placed within the tip element 11 and reaches to the very front of the tip element 11, as shown in Fig. 3A. In this state opening 110 on the circumference of the tip element 11 are closed off by the probe device 22, such that body fluid may not enter into a channel opening 220 of the probe device 22.

Once the tube 10 has reached its desired position (or at least is assumed to have reached its desired position) the guide wire 10 may be moved within the tube 10 in a pulling direction P1 such that at least one of the openings 110 comes into alignment with the channel opening 220 within the probe device 22, upon which body fluid may enter into the channel opening 220 and may come into contact with an indicator device 221 placed on the probe device 22. By subjecting the indicator device 221 to the body fluid, the body fluid may be tested in particular for specific substances which are characteristic for the body fluid in the patient's stomach 32.

The indicator device 221 may, in one embodiment, comprise a pH-sensitive substance impregnated or mixed with an ester compound. If the ester compound is subjected to human gastric lipase, which is commonly present in the fluid contained in the patient's stomach 32, carboxylic acid is released by the action of the human gastric lipase enzyme on the ester compound, which will lead to a decrease in pH, which can be registered by the pH-sensitive substance and will for example induce a color change of the pH-sensitive substance.

Upon withdrawing the guide wire 20 together with the probe device 22 from the tube 10, the indicator device 221 can be visually inspected, and in this way the presence of human gastric lipase in the body opening which the tube 10 has entered can be confirmed.

In another embodiment which does not form part of the invention, shown in Fig. 4A and 4B, a probe device 22 arranged on a guide wire 20 is received, when inserting the tube 10 into the patient 3, in the tube 10 such that the probe device 22 is concealed within a tip element 11 arranged on the tube 10, as it is shown in Fig. 4A.

The tip element 11 comprises, at its far end, an exit opening 111 through which the probe device 22 may be moved in a pushing direction P2, as indicated in Fig. 4B, such that an indicator device 221 for example of the kind described above may come into contact with body fluid in the body opening the tube 10 has entered. Upon retracting the guide wire 20 in the pulling direction P1 from the tube 10, the probe device 22 may be visually inspected in order to confirm correct placement of the tube 10 in the desired body opening.

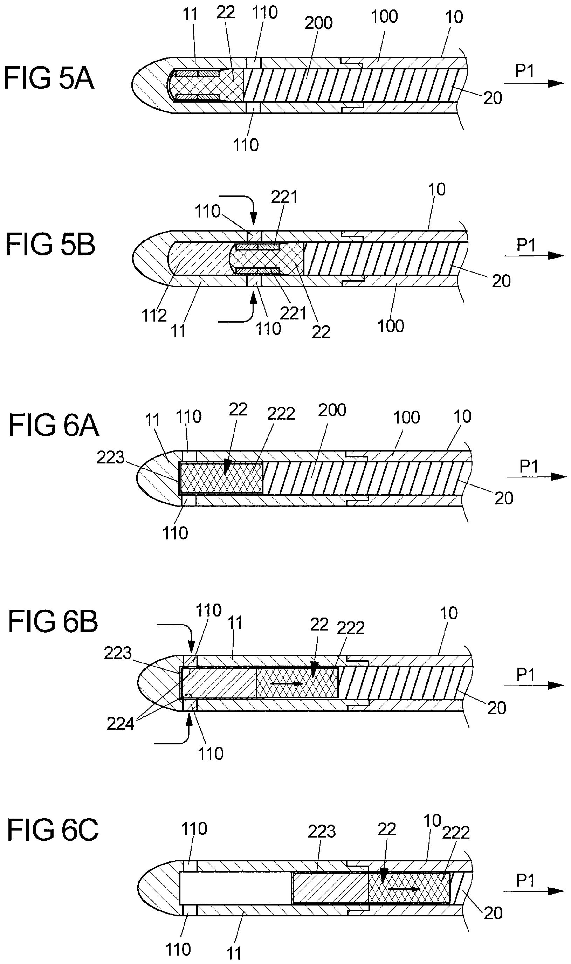

In yet another embodiment shown in Fig. 5A and 5B, a probe device 22 is arranged on a guide wire 20 and, when placing the tube 10 in the patient 3, is concealed in a tip element 11 arranged on the tube 10.

When placing the tube 10 in the patient 3 openings 110 on the outer circumference of the tip element 11 are closed off by means of the guide wire 20, as shown in Fig. 5A. By retracting the guide wire 20 in the pulling direction P1, as shown in Fig. 5B, the openings 110 may be uncovered such that body fluid may flow into an inner space 112 of the tip element 11 and may come into contact with an indicator device 221 arranged on the probe device 22, which for example may be of the kind as described above.

Upon fully retracting the guide wire 20 from the tube 10 the indicator device 221 may be visually inspected in order to confirm correct placement of the tube 10 within the patient 3.

In yet another embodiment shown in Fig. 6A to 6C, a probe device 22 arranged on a guide wire 20 comprises a probing chamber 223 slidingly arranged on the distal end 200 of the guide wire 20 and resting within a tip element 11 arranged on the tube 10 when inserting the tube 10 into the patient 3. The probing chamber 223 comprises openings 224 aligned with openings 110 of the tip element 11, wherein such openings 224 are closed off by means of an insertion piece 222 connected to the guide wire 20 when sliding the tube 10 into the patient 3, as it is shown in Fig. 6A.

Once the tube 10 has reached its presumed position, by withdrawing the guide wire 20 the insertion piece 222 is moved with respect to the probing chamber 223 such that the openings 224 are opened towards the openings 110 of the tip element 11 and, by the movement of the insertion piece 222, body fluid is drawn into the probing chamber 223, as it is shown Fig. 6B.

By withdrawing the guide wire 20 together with the probing chamber 223, as shown in Fig. 6C, a small volume sample of body fluid is taken, which may be tested outside of the patient's body by a testing device as for example described in US 8,696,567.

The probing chamber 223 together with the insertion piece 222 functions as a miniature syringe in order to draw body fluid into the probing chamber 223. The probing chamber 223, when inserting the tube 10 in the patient 3, rests in the tip element 11 and is held in the tip element 11 for example by a (releasable) form locking engagement of edge sections surrounding the openings 224 with the openings 110 in the tip element 11. When withdrawing the guide wire 20 (after the tube 10 has reached its presumed position), the probing chamber 223 is at first held in place by the engagement with the openings 110, such that the insertion piece 222 (which is fixedly connected to the guide wire 20) slides with respect to the probing chamber 223. Due to the movement of the insertion piece 222 with respect to the probing chamber 223 body fluid is drawn into the probing chamber 223. Once the displacement of the insertion piece 222 with respect to the probing chamber 223 has reached a predefined maximum, the probing chamber 223 is moved together with the insertion piece 222 connected to the guide wire 20 by overcoming the form locking engagement (which is released when the pulling force on the probing chamber 223 exceeds a maximum force of the form locking engagement) and is withdrawn, together with the guide wire 20, from the tube 10.

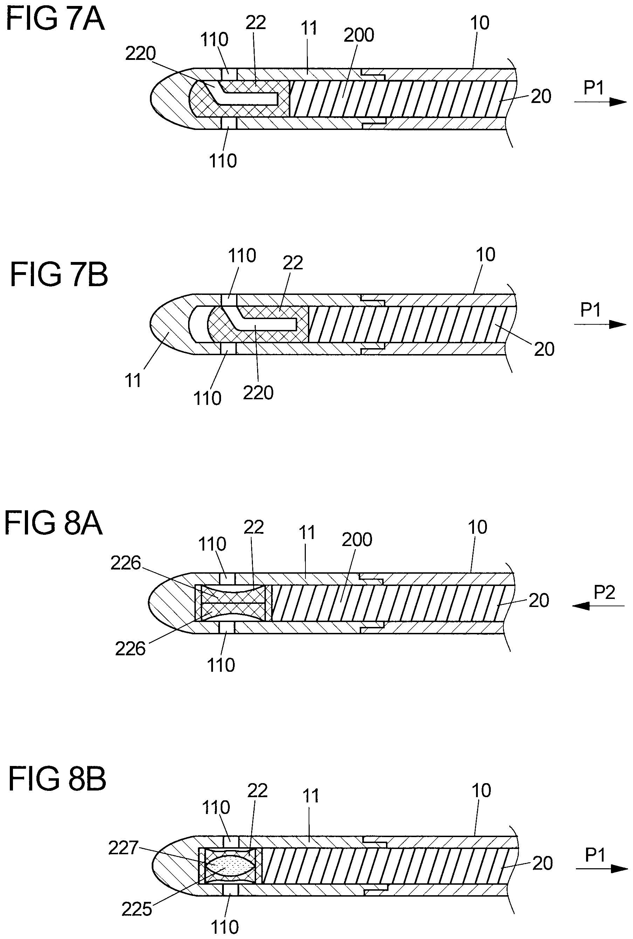

In yet another embodiment shown in Fig. 7A and 7B a probe device 22 arranged on a guide wire 20 comprises a chamber device in the shape of a channel opening 220, which, while inserting the tube 10 into the patient 3, is not aligned with openings 110 on the outer circumference of a tip element 11 arranged on the tube 10, as it is shown in Fig. 7A. Once the tube 10 has reached its position, the guide wire 20 may be retracted from the tube 10 by pulling it in the pulling direction P1, upon which the channel opening 220 comes into alignment with one of the openings 110 on the tip element 11, as shown in Fig. 7B, such that body fluid may enter into the channel opening 220 in order to take a sample of body fluid for testing outside of the patient's body.

In yet another embodiment shown in Fig. 8A and 8B a probe device 22 arranged on a guide wire 20 comprises two (elastically or plastically) deformable concealing elements 226 receiving a sponge element 225 therebetween. In a non-deformed state, shown in Fig. 8A, the sponge element 225 is concealed in between the concealing elements 226 such that it is not subjected with body fluid entering through openings 110 in a tip element 11 connected to the tube 10. By pushing on the guide wire 20 relative to the tube 10, once the tube 10 has reached its position in the patient 3, the concealing elements 226 can be deformed as it is shown in Fig. 8B, such that a reception opening 227 in between the concealing elements 226 is uncovered and body fluid may come into contact with the sponge element 225 received in between the concealing elements 226. The guide wire 20 may then be retracted from the tube 10 in order to perform a testing of the sample of body fluid stored in the sponge element 225.

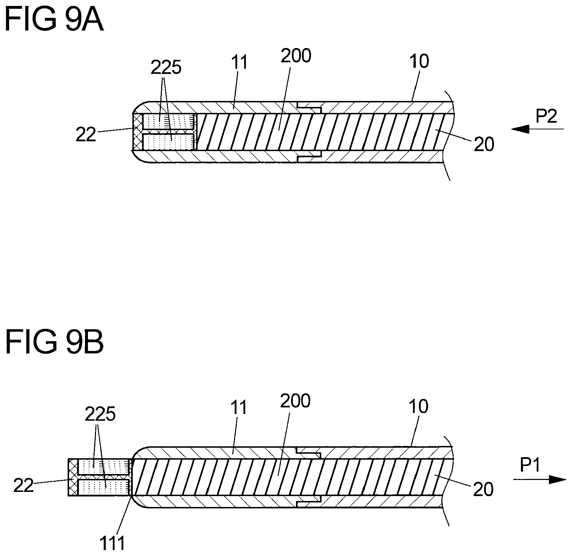

In yet another embodiment, which does not form part of the invention, shown in Fig. 9A and 9B sponge elements 225 are arranged on a probe device 22 connected to a guide wire 20. The guide wire 20, when inserting a tube 10 into a patient 3, is received and concealed within the tube 10 and a tip element 11 connected to the tube 10. Once the tube 10 has reached its position, the probe device 22 may be moved in a pushing direction P2 relative to the tube 10 out of an exit opening 111 at the very front of the tip element 11 in order to protrude from the tip element 11, as illustrated in Fig. 9B. The sponge elements 225 hence our subjected to body fluid such that, by withdrawing the guide wire 20 together with the probe device 22 from the tube 10 in a pulling direction P1, a sample is taken and may be tested outside of the patient's body.

The idea underlying the invention is not limited to the embodiments described above, but may be implemented in an entirely different fashion in entirely different embodiments that fall within the scope of the appended claims. For example, a probe device may be constituted to take a sample and in addition may comprise an indicator device to perform a testing directly within the body opening the tube reaches into.

Although described with regard to enteral feeding into the gastro-intestinal tract, a tube device of the kind described herein may also be used for other purposes, such as gastro-intestinal decompression or for diagnostic purposes. A tube device of the kind described herein may in particular be used for all sorts of gastro-intestinal actions.

### List of Reference Numerals

- 1: Tube device
- 10: Tube
- 100: Distal tube end
- 101: Proximal tube end
- 11: Tip
- 110: Opening
- 111: Exit opening
- 112: Inner space
- 2: Guide device
- 20: Guide wire
- 200: Distal end
- 22: Probe device
- 220: Probe opening
- 221: Indicator device
- 222: Insertion piece
- 223: Probing chamber
- 224: Opening
- 225: Sponge element
- 226: Concealing elements
- 227: Reception opening
- 3: Patient
- 30: Nose
- 31: Esophagus
- 32: Stomach
- P1: Pulling direction
- P2: Pushing direction

## Claims

1. Tube device (1) for administering a medical fluid to a patient (P), comprising:
- a tube (10) comprising at least one opening (110) constituted to allow body fluid to enter into the tube (10), and
- a guide device (2) having a guide wire (20) which is insertable into the tube (10) for introducing the tube (10) into a body opening (30-32) of a patient (3), wherein the guide wire (20) comprises a probe device (22) for testing or obtaining a sample of a body fluid present in the body opening (30-32),
**characterized in**
**that** the guide wire (20) in a first position is placed in the tube (10) to close off the opening (110) such that body fluid is prevented from entering into the tube (10) and in a second position is placed in the tube (10) to uncover the opening (110) such that body fluid is allowed to enter into the tube (10) to interact with the probe device (22).

2. Tube device (1) according to claim 1, **characterized in that** the probe device (22) is placed at a distal end (200) of the guide wire (20), the guide wire (22) being inserted into the tube (10) with the distal end (200).

3. Tube device (1) according to one of the preceding claims, **characterized in that** the probe device (22) comprises at least one indicator device (222) which is constituted, upon interaction with body fluid, to indicate at least one property of the body fluid.

4. Tube device (1) according to claim 3, **characterized in that** the at least one indicator device (222) is constituted to indicate the presence of at least one substance in the body fluid.

5. Tube device (1) according to claim 4, **characterized in that** the indicator device (222) comprises at least one of an ester compound and a pH-sensitive substance for obtaining an indication of the presence of a hydrolytic enzyme, in particular human gastric lipase, in the body fluid.

6. Tube device (1) according to one of the preceding claims, **characterized in that** the probe device (22) comprises a probing chamber (223) for receiving a sample of body fluid.

7. Tube device (1) according to claim 6, **characterized in that** the guide wire (20) comprises an insertion piece (222) movable with respect to the probing chamber (223) for delivering body fluid into the probing chamber (223).

8. Tube device (1) according to claim 7, **characterized in that** the probe device (22) comprises a sponge element (225) for storing body fluid.

9. Tube device (1) according to one of claims 6 to 8, **characterized in that** the probe device (22) comprises at least one deformable concealing element (226) which in a non-deformed state conceals an reception opening (227) and is deformable to open the reception opening (227) for receiving body fluid.

## Patentansprüche

1. Schlauchvorrichtung (1) zum Verabreichen einer medizinischen Flüssigkeit an einen Patienten (P), umfassend:
- einen Schlauch (10) umfassend mindestens eine Öffnung (110), die so ausgelegt ist, dass Körperflüssigkeit in den Schlauch (10) eintreten kann, und
- eine Führungsvorrichtung (2) mit einem Führungsdraht (20), der in den Schlauch (10) eingeführt werden kann, um den Schlauch (10) in eine Körperöffnung (30-32) eines Patienten (3) einzuführen, wobei der Führungsdraht (20) eine Sondenvorrichtung (22) zum Testen oder Erhalten einer Probe einer in der Körperöffnung (30-32) vorhandenen Körperflüssigkeit umfasst,
**dadurch gekennzeichnet,**
**dass** der Führungsdraht (20) in einer ersten Position in den Schlauch (10) eingebracht wird, um die Öffnung (110) so zu verschließen, dass verhindert wird, dass Körperflüssigkeit in den Schlauch (10) eindringt, und in einer zweiten Position in den Schlauch (10) eingebracht wird, um die Öffnung (110) so freizulegen, dass Körperflüssigkeit in den Schlauch (10) eintreten kann, um mit der Sondenvorrichtung (22) zu interagieren.

2. Schlauchvorrichtung (1) nach Anspruch 1, **dadurch gekennzeichnet, dass** die Sondenvorrichtung (22) an einem distalen Ende (200) des Führungsdrahtes (20) angeordnet ist, wobei der Führungsdraht (22) mit dem distalen Ende (200) in den Schlauch (10) eingeführt wird.

3. Schlauchvorrichtung (1) nach einem der vorstehenden Ansprüche, **dadurch gekennzeichnet, dass** die Sondenvorrichtung (22) mindestens eine Anzeigevorrichtung (222) umfasst, die bei Interaktion mit Körperflüssigkeit ausgelegt ist, um mindestens eine Eigenschaft der Körperflüssigkeit anzuzeigen.

4. Schlauchvorrichtung (1) nach Anspruch 3, **dadurch gekennzeichnet, dass** die mindestens eine Anzeigevorrichtung (222) so ausgelegt ist, dass sie das Vorhandensein mindestens einer Substanz in der Körperflüssigkeit anzeigt.

5. Schlauchvorrichtung (1) nach Anspruch 4, **dadurch gekennzeichnet, dass** die Indikatorvorrichtung (222) mindestens eine Esterverbindung und eine pH-empfindliche Substanz umfasst, um einen Hinweis auf das Vorhandensein eines hydrolytischen Enzyms, insbesondere menschlicher Magenlipase, in der Körperflüssigkeit zu erhalten.

6. Schlauchvorrichtung (1) nach einem der vorstehenden Ansprüche, **dadurch gekennzeichnet, dass** die Sondenvorrichtung (22) eine Sondenkammer (223) zum Aufnehmen einer Körperflüssigkeitsprobe umfasst.

7. Schlauchvorrichtung (1) nach Anspruch 6, **dadurch gekennzeichnet, dass** der Führungsdraht (20) ein Einführstück (222) umfasst, das in Bezug auf die Sondierungskammer (223) beweglich ist, um Körperflüssigkeit in die Sondierungskammer (223) abzugeben.

8. Schlauchvorrichtung (1) nach Anspruch 7, **dadurch gekennzeichnet, dass** die Sondenvorrichtung (22) ein Schwammelement (225) zum Speichern von Körperflüssigkeit umfasst.

9. Schlauchvorrichtung (1) nach einem der Ansprüche 6 bis 8, **dadurch gekennzeichnet, dass** die Sondenvorrichtung (22) mindestens ein verformbares Verdeckelement (226) umfasst, das in einem nicht verformten Zustand eine Aufnahmeöffnung (227) verdeckt und verformbar ist, um die Aufnahmeöffnung (227) zum Aufnehmen von Körperflüssigkeit zu öffnen.

## Revendications

1. Dispositif tubulaire (1) pour l'administration d'un fluide médical à un patient (P), comprenant :
- un tube (10) comprenant au moins un orifice (110) constitué pour permettre au fluide corporel d'entrer dans le tube (10), et
- un dispositif de guidage (2) ayant un fil de guidage (20) qui est insérable dans le tube (10) pour l'introduction du tube (10) dans un orifice corporel (30-32) d'un patient (3), dans lequel le fil de guidage (20) comprend un dispositif de sonde (22) pour le test ou l'obtention d'un échantillon d'un fluide corporel présent dans l'orifice corporel (30-32),
**caractérisé en**
**ce que** le fil de guidage (20) dans une première position est placé dans le tube (10) pour fermer l'orifice (110) de sorte à empêcher le fluide corporel d'entrer dans le tube (10) et dans une deuxième position est placé dans le tube (10) pour découvrir l'orifice (110) de sorte à permettre au fluide corporel d'entrer dans le tube (10) pour interagir avec le dispositif de sonde (22).

2. Dispositif tubulaire (1) selon la revendication 1, **caractérisé en ce que** le dispositif de sonde (22) est placé à une extrémité distale (200) du fil de guidage (20), le fil de guidage (22) étant inséré dans le tube (10) avec l'extrémité distale (200).

3. Dispositif tubulaire (1) selon l'une des revendications précédentes, **caractérisé en ce que** le dispositif de sonde (22) comprend au moins un dispositif indicateur (222) qui est constitué, lors de l'interaction avec le fluide corporel, pour indiquer au moins une propriété du fluide corporel.

4. Dispositif tubulaire (1) selon la revendication 3, **caractérisé en ce que** l'au moins un dispositif indicateur (222) est constitué pour indiquer la présence d'au moins une substance dans le fluide corporel.

5. Dispositif tubulaire (1) selon la revendication 4, **caractérisé en ce que** le dispositif indicateur (222) comprend au moins l'un parmi un composé ester et une substance sensible au pH pour l'obtention d'une indication de la présence d'une enzyme hydrolytique, en particulier la lipase gastrique humaine, dans le fluide corporel.

6. Dispositif tubulaire (1) selon l'une des revendications précédentes, **caractérisé en ce que** le dispositif de sonde (22) comprend une chambre de sondage (223) pour la réception d'un échantillon de fluide corporel.

7. Dispositif tubulaire (1) selon la revendication 6, **caractérisé en ce que** le fil de guidage (20) comprend une pièce d'insertion (222) mobile par rapport à la chambre de sondage (223) pour la délivrance de fluide corporel dans la chambre de sondage (223).

8. Dispositif tubulaire (1) selon la revendication 7, **caractérisé en ce que** le dispositif de sonde (22) comprend un élément en éponge (225) pour le stockage de fluide corporel.

9. Dispositif tubulaire (1) selon l'une des revendications 6 à 8, **caractérisé en ce que** le dispositif de sonde (22) comprend au moins un élément d'occultation déformable (226) qui occulte dans un état non déformé un orifice de réception (227) et est déformable pour ouvrir l'orifice de réception (227) pour la réception du fluide corporel.
